Europäisches Patentamt

European Patent Office

Office européen des brevets

(11) Publication number: **0 520 547 A2**

# EUROPEAN PATENT APPLICATION

(21) Application number: 92201742.1

(22) Date of filing: 12.06.92

(51) Int. Cl.5: **A61L 9/04**, A61L 9/01

(30) Priority: **13.06.91 EP 91201502**

(43) Date of publication of application:
**30.12.92 Bulletin 92/53**

(84) Designated Contracting States:
**AT BE CH DE DK ES FR GB GR IT LI LU MC NL PT SE**

(71) Applicant: Sara Lee/DE N.V.
Keulsekade 143
NL-3532 AA Utrecht(NL)

(72) Inventor: de Groot, Anthonius Daniel
Waterkers 27
NL-2804 PH Gouda(NL)

(74) Representative: Smulders, Theodorus A.H.J.,
Ir. et al
Vereenigde Octrooibureaux Nieuwe Parklaan 97
NL-2587 BN 's-Gravenhage(NL)

(54) Air freshener composition.

(57) This invention relates to an air freshener composition comprising, based upon the weight of the composition
a) at least 30 wt.% of at least one $C_1$-$C_6$ alcohol,
b) at least 5 wt.% of at least one glycol selected from the group consisting of ethylene glycol monoethyl ether, ethylene glycol monomethyl ether, propylene glycol monoethyl ether, propylene glycol monomethyl ether, dipropylene glycol monoethyl ether and dipropylene glycol monomethyl ether, and wherein the amount of a and b together is at least 40 wt.%.
c) 0,5 to 20 wt.% of at least one perfume, and
d) water up to 100 wt.%.

The present invention is directed to an air freshener composition suitable for use in air freshener systems based upon liquid air fresheners.

Conventionally there are two types of liquid air freshener systems. The first system is based thereon that the clear liquid, containing an active ingredient, such as a perfume is sprayed into the air. This can for example be done from a pressurized aerosol container, or from a container where the pressure is applied manually through a pump or because the bottle is flexible. The other system is based on a wick. A container, such as a bottle, contains the air freshener liquid which is evaporated through the wick.

The air freshener for the first system usually consists of 97.5% of ethanol and the remainder being perfume. The system based upon the wick usually contains a mixture of alcohol, water, perfume and solubilizer. In view of economical, environmental and fire considerations, it would be better if the amount of alcohol in the freshener composition could be reduced, without detracting from the advantages of the use thereof, such as the solubility of the perfume and the problems inherent to the use of higher amounts of solubilizer.

With respect to the solubilizer it can be remarked that this is generally a compound which precipitates on either the surface of the wick, if a wick system is used, or in the room where the vaporizer is used, in the situation where a spray system is used.

Decreasing the amount of alcohol would necessitate an increase in the amount of solubilizer, as otherwise the system would become unstable.

It is thus an object of the invention to provide an air freshener composition that can be used in both the spraying system and the wick system, with a decreased amount of alcohol, and without any substantive use of an solubilizer.

The air freshener composition of the present invention comprises, based upon the weight of the composition,

a) at least 30 wt.% of at least one $C_1$-$C_6$ alcohol,
b) at least 5 wt.% of at least one glycol selected from the group consisting of ethylene glycol monoethyl ether, ethylene glycol monomethyl ether, propylene glycol monoethyl ether, propylene glycol monomethyl ether, dipropylene glycol monoethyl ether and dipropylene glycol monomethyl ether, and wherein the amount of a and b together is at least 40 wt.%.
c) 0,5 to 20 wt.% of at least one perfume, and
d) water up to 100 wt.%.

According to a preferred embodiment of the present invention, the air freshener composition consists only of those components, optionally in combination with some colouring agent.

The essential aspect of the present invention is the combined use of at least one $C_1$-$C_6$ alcohol, with at least one glycol selected from the group enumerated hereinabove. Surprisingly it has been found that this combined use results in a liquid air freshener composition with has a very good evaporation behaviour, does not cause precipitation on the wick or on the surfaces in the room where it is used, and has a flash point above 21°C.

In the air freshener composition according to the present invention, the use of solubilizer is not necessary, and is preferably prevented. More in particular the amount of solubilizer is below 1,0 wt.% based on the weight of the composition, preferably less than 0,1 wt.% and in the most preferred situation no solubilizer is added at all. Of course it has to be taken into account that some perfume solutions contain a small amount of emulisifier.

The $C_1$-$C_6$ alcohol used in accordance with the present invention is preferably ethanol, although methanol and propanol can also be used. The higher than $C_3$ alcohols should preferably be branched alcohols. Most preferred is ethanol.

Of the glycols the propylene glycol monomethyl ether is the preferred component, as this shows the most suitable evaporation and dissolution behaviour.

The amounts of the various components can be varied within the ranges given in the claim, wherein it is remarked that it is of importance that the amount of the alcohol and the glycol are at least 40 wt.%. The upper limit of the combined components is preferably 75 wt.%, although a range of 50 to 60 wt.% is preferred. The ratio of the two components can be varied to a degree within the ranges given, whereby it is remarked that the amount of ethanol should preferably be not be above 55 wt.% based upon the weight of the composition, as otherwise the flash point of the composition would become too high. In case this would not be a problem, it may of course be possible to increase the amount of alcohol.

As perfume, the various well-known perfumes in this art can be used. The amount thereof can range from 0.5 to 20 wt.% of the composition, more preferably from 1 to 10 wt.%. The amount of perfume will depend upon various aspects, such as the strength thereof, the required application and the solubility thereof in the system.

According to another embodiment of the invention the perfume can be replaced in whole or in part by an insecticide.

The air freshener composition according to the invention can be prepared in any known manner by mixing the various components in the required ratio. Of course one has to take into account the stability and dissolution aspects thereof, which can be ascertained easily by routine experiments.

The invention is also directed to a container wherein an amount of the air freshener composition as disclosed hereinabove is present.

The invention is now elucidated on the basis of an example, which example is not intended to limit the application in any way.

## Example

An air freshener composition was prepared from 50 wt.% of ethanol, 35 wt.% of water, 10 wt.% of propylene glycol monomethyl ether and 5 wt.% of perfume.

Due to the evaporation behaviour the air freshener composition of this example could be used in a wick having a small evaporation surface. As the composition does not contain any components that are not volatile, the sprayed composition did not result in any residue on a surface in the room where it was used.

## Claims

1. Air freshener composition comprising, based upon the weight of the composition
   a) at least 30 wt.% of at least one $C_1$-$C_6$ alcohol,
   b) at least 5 wt.% of at least one glycol selected from the group consisting of ethylene glycol monoethyl ether, ethylene glycol monomethyl ether, propylene glycol monoethyl ether, propylene glycol monomethyl ether, dipropylene glycol monoethyl ether and dipropylene glycol monomethyl ether, and wherein the amount of a and b together is at least 40 wt.%.
   c) 0,5 to 20 wt.% of at least one perfume, and
   d) water up to 100 wt.%.

2. Air freshener composition according to claim 1, consisting of, based upon the weight of the composition
   a) at least 30 wt.% of at least one $C_1$-$C_6$ alcohol,
   b) at least 5 wt.% of at least one glycol selected from the group consisting of ethylene glycol monoethyl ether, ethylene glycol monomethyl ether, propylene glycol monoethyl ether, propylene glycol monomethyl ether, dipropylene glycol monoethyl ether and dipropylene glycol monomethyl ether, and wherein the amount of a and b together is at least 40 wt.%.
   c) 0,5 to 20 wt.% of at least one perfume, and
   d) balance water.

3. Air freshener composition according to claim 1 or 2, wherein the alcohol is ethanol.

4. Air freshener composition according to claim 1-3, wherein the glycol is propylene glycol monomethyl ether.

5. Air freshener composition according to claim 1-4, wherein the amount of perfume ranges from 1 to 10 wt.%.

6. Air freshener composition according to claim 1-5, wherein the composition contains substantially no solubilizer.

7. Air freshener composition according to claim 1-6, wherein the perfume is replaced in whole or in part by at least one insecticide.

8. Air freshener system comprising a container and an air freshener composition according to claims 1-7.